(19) Europäisches Patentamt
European Patent Office
Office européen des brevets

(11) Numéro de publication: **0 381 563 A1**

(12) **DEMANDE DE BREVET EUROPEEN**

(21) Numéro de dépôt: **90400229.2**

(22) Date de dépôt: **26.01.90**

(51) Int. Cl.5: **C07C 69/743, C07C 61/40, A01N 53/00**

Claim for the following Contracting State: ES.

(30) Priorité: **30.01.89 FR 8901110**

(43) Date de publication de la demande: **08.08.90 Bulletin 90/32**

(84) Etats contractants désignés: **AT BE CH DE ES FR GB IT LI LU NL**

(71) Demandeur: **ROUSSEL-UCLAF**
**35, boulevard des Invalides**
**F-75007 Paris(FR)**

(72) Inventeur: **Demassey, Jacques**
**Allée Saint Jacques Chalifert**
**F-77144 Montevrain(FR)**
Inventeur: **Demoute, Jean-Pierre**
**Canta Cigalo**
**F-13390 Auriol(FR)**
Inventeur: **Tessier, Jean**
**30, Rue Jean Moulin**
**F-94300 Vincennes(FR)**

(74) Mandataire: **Tonnellier, Marie-José et al**
**111, route de Noisy B.P. no 9**
**F-93230 Romainville(FR)**

(54) **Nouveaux dérivés de l'acide 2,2-diméthyl 3-(2-monohaloéthényl) cyclopropane carboxylique, leur procédé de préparation et leur application comme pesticides.**

(57) L'invention a pour objet les composés de formule (I) :

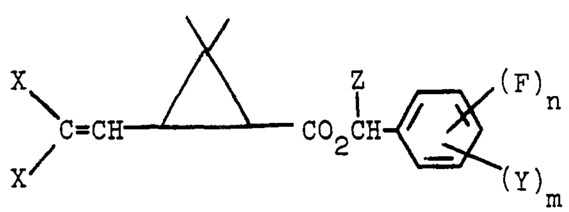

(I)

dans lesquels :
- X représente un atome d'halogène ;
- Z représente un atome d'hydrogène, un radical $CH_3$, C≡N ou C≡CH ;
- n représente un nombre entier pouvant varier de 1 à 5 ;
- m représente le nombre 5-n ;
- Y représente un atome d'hydrogène, un atome d'halogène, un radical $CH_2$-C≡N, un radical hydroxy, un radical alkyle, un radical nitrile, un radical

$\overset{O}{\overset{\|}{C}}$ -O-alkyle,

$\overset{O}{\overset{\|}{C}}$ -alkyle, $(CH_2)m'$ O-alkyle, $(CH_2)m'$ S alkyle, $(CH_2)m'$ N-$(alkyle)_2$ dans lequel m' représente 1, 2, 3 ou 4 et renfermant jusqu'à 12 C, un radical Si $(alkyle)_3$, alkyle ($C_{1-8}$), un radical O-aryle ou -$(CH_2)m'$-aryle, aryle ($C_{6-14}$),
leur procédé de préparation, leur application comme pesticides et les compositions les renfermant.

EP 0 381 563 A1

## Nouveaux dérivés de l'acide 2,2-diméthyl 3-(2-monohaloéthényl) cyclopropane carboxylique, leur procédé de préparation et leur application comme pesticides.

La présente invention concerne des dérivés de l'acide 2,2-diméthyl 3(2-monohalo éthényl) cyclopropane carboxylique, leur procédé de préparation, leur application comme pesticides et les compositions les renfermant.

L'invention a pour objet sous toutes les formes stéréoisomères possibles, ainsi que les mélanges de ces stéréoisomères les composés de formule (I) :

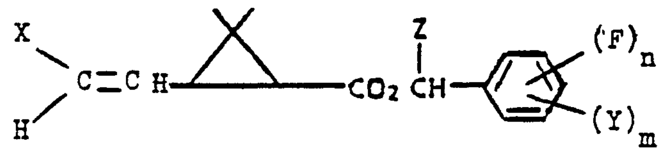

(I)

dans laquelle :

- X représente un atome d'halogène ;
- Z représente un atome d'hydrogène, un radical $CH_3$, C≡N ou C≡CH ;
- n représente un nombre entier pouvant varier de 1 à 5 ;
- m représente le nombre 5-n ;
- Y représente un atome d'hydrogène, un atome d'halogène, un radical $CH_2$ C≡N, un radical hydroxy, un radical alkyle linéaire, ramifié, saturé ou insaturé éventuellement substitué renfermant jusqu'à 8 atomes de carbone, un radical nitrile un radical

$\overset{O}{\overset{\|}{C}}$ -O-alkyle,

$\overset{O}{\overset{\|}{C}}$ -alkyle, $(CH_2)m'$ O-alkyle, $(CH_2)m'$ S alkyle, $(CH_2)m'$ N-$(alkyle)_2$, dans lequel m′ représente le nombre 0, 1, 2, 3 ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou $-(CH_2)m'$-aryle, aryle représentant un radical aryle renferment jusqu'à 14 atomes de carbone, le ou ls substituants Y identiques ou différents, pouvant être en position quelconque sur le noyau phényle.

Lorsque Y représente un atome d'halogène, il s'agit de préférence d'un atome de fluor.

Dans la définition de Y, lorsqu'il s'agit de radicaux alkyles, il s'agit de préférence de radicaux méthyle, éthyle, propyle, isopropyle, n-butyle ou tertbutyle.

Lorsque Y représente un radical alkyle insaturé, il s'agit d'un radical éthylénique comme par exemple éthényle, propényle ou propadiényle ou d'un radical acétylénique comme par exemple le radical éthynyle ou propynyle.

Lorsque Y représente un radical alkyle substitué par un ou plusieurs groupements fonctionnels, on entend de préférence par groupement fonctionnel les atomes d'halogène, tels que le fluor ou le brome ; Y peut être par exemple le radical $CF_3$.

Lorsque Y représente un radical O-aryle ou $(CH_2)m'$ aryle on entend de préférence par aryle le radical phényle.

Lorsque Y représente un radical $(CH_2)m'$ O-alkyle, ou $(CH_2)m'$ S alkyle, il s'agit de préférence du radical $OCH_3$ ou $SCH_3$.

L'invention a tout spécialement pour objet les composés répondant à la formule ($I_A$) :

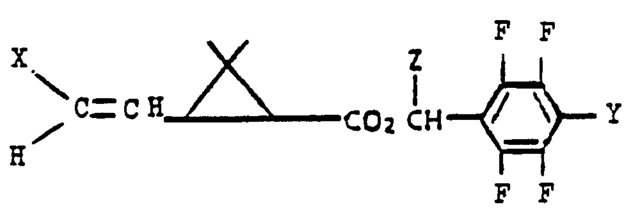

($I_A$)

dans lesquels X, Z et Y conservent la même signification que précédemment.

Parmi ces composés de formule ($I_A$) on peut citer tout spécialement les composés dans lesquels :

- Y représente un atome d'hydrogène, un atome d'halogène, un radical hydroxy un radical alkyle linéaire, ramifié, saturé ou insaturé éventuellement substitué renfermant jusqu'à 8 atomes de carbone, un radical nitrile, un radical $(CH_2)m'$ O-alkyle, $(CH_2)m'$ S alkyle, $(CH_2)m'$ N-$(alkyle)_2$, dans lequel m′ représente le nombre 0, 1, 2, 3 ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone.

Parmi les composés préférés de l'invention, on peut citer :

- les composés de formule (I) ou ($I_A$) dans lesquels Z représente un atome d'hydrogène ;
- les composés de formule (I) ou ($I_A$) dans lesquels X représente un atome de fluor, de chlore ou de brome ;
- les composés de formule (I) ou ($I_A$) dans lesquels Y représente un atome d'hydrogène, ou encore un atome de fluor, ou encore un radical alkyle linéaire saturé ou insaturé renfermant jusqu'à 4 atomes de carbone, par exemple un radical méthyle ou bien un radical propynyle.

Parmi les composés préférés, on peut citer particulièrement les composés de structure 1Rcis.

L'invention a tout spécialement pour objet les composés dont la préparation est donnée ci-après dans la partie expérimentale et notamment les composés des exemples 1, 2, 3, 14, 30, 46, 47 et 49.

L'invention a également pour objet un procédé de préparation caractérisé en ce que l'on soumet en présence d'un agent d'esterification un acide de formule :

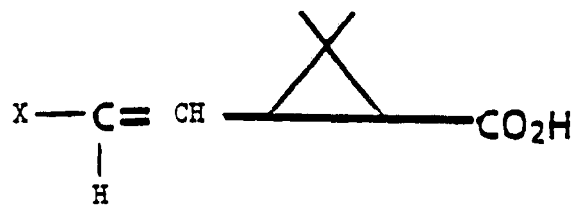

(II)

dans laquelle X conserve sa signification précédente à l'action d'un alcool de formule :

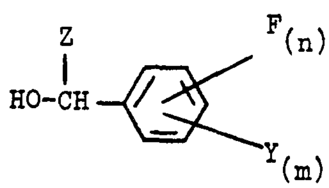

(III)

dans laquelle Y, Z, n et m conservent leur signification précédente pour obtenir le composé de formule (I) correspondant.

L'acide de formule (II) de structure 1R trans dans lequel X est un atome de chlore est un produit qui peut être préparé à partir de l'ester terbutylique correspondant décrit dans le brevet français 2185612. L'acide correspondant de structure 1Rcis est préparé comme l'acide de structure 1R trans à partir de produit de départ de structure 1Rcis. Les autres acides de formule (II) sont des produits nouveaux et constituent en eux-mêmes un objet de la présente invention : leur prépration donnée ci-après dans la partie expérimentale, peut être résumée par les schémas suivants :

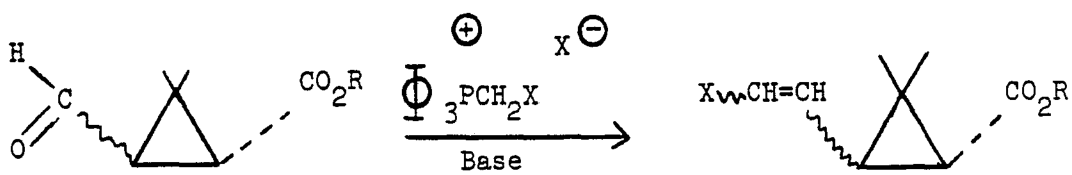


avec R = H ou reste d'alcool.

Si R est un reste d'alcool on soumet le produit de formule final à l'action d'une hydrolyse acide ou basique.

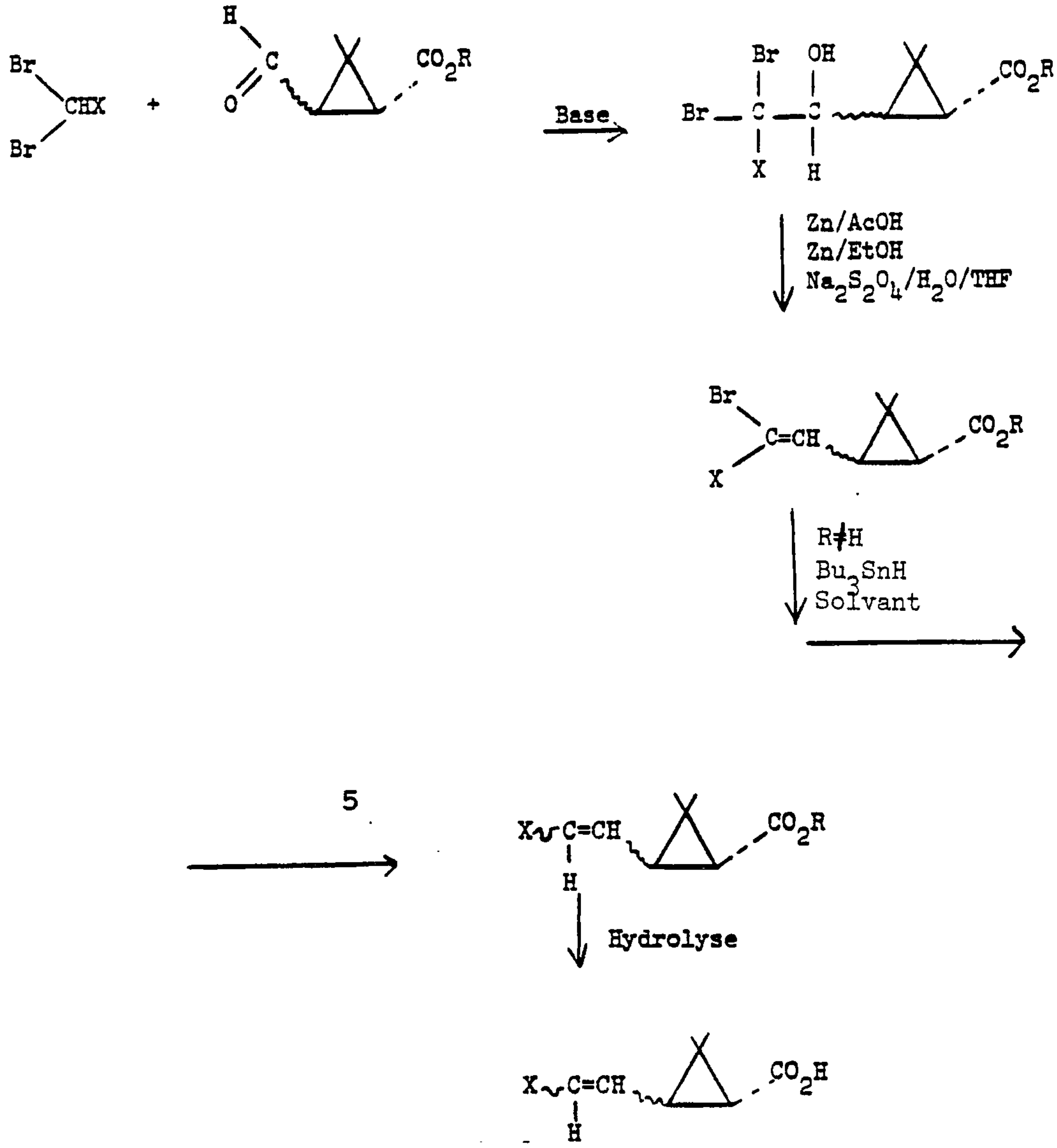


Les composés de formule (III) sont des composés connus d'une manière générale ; ils peuvent être préparés par exemple selon les procédés décrits dans la demande de brevet européen 0031199, dans les brevets américains 4370346, 4405640, dans le brevet anglais 2171994 ou dans British Crop Protection Conference Pest and Desease 1986 page 199, ou encore dans la demande de brevet européen 0281439.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour contre les insectes et autres parasites du sol, par exemple les coléoptères, comme DIABROTICA, les taupins et les vers blancs, les myriapodes comme les scutigerelles et les blaniules, et les diptères comme les Cecydomies et les lépidoptères comme les noctuelles terricoles.

Ils sont utilisés à des doses comprises entre 10 g et 300 g de matière active à l'hectare.

Les produits de formule I peuvent également être utilisés pour lutter contre les insectes dans les locaux, pour lutter notamment contre les mouches, les moustiques et les blattes.

Les produits de formule I sont, de plus, photostables et sont peu toxiques pour les mammifères.

L'ensemble de ces propriétés fait que les produits de formule I correspondent parfaitement aux exigences de l'industrie agrochimique moderne : ils permettent de protéger les récoltes tout en préservant l'environnement.

Les produits de formule I peuvent aussi être utilisés pour lutter contre les acariens et les nématodes parasites des végétaux.

Les composés de formule I peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule I définis ci-dessus et notamment les produits de formule (I) des exemples 1, 2, 3, 14, 30, 46, 47 et 49.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives un mélange. Le véhicule utilisé peut être un liquide, tel que l'eau, l'alcool, les hydrocarbures ou autres solvents organiques, une huile minérale, animale ou végétale, une poudre telle que le talc, les argiles, les silicates, le kieselguhr ou un solide combustible.

Les compositions insecticides selon l'invention contiennent de préférence de 0.005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0.03 à 1 % en poids.

Dans le cas où l'on utilise un support fibreux incombustible, la dose de matière active peut alors être, par exemple, de 0.03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0.03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de

0.05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo /2,2-1/ 5-heptène-2,3-dicarboximide, ou le pipéronyl-bis-2-(2′-n-butoxy éthoxy) éthyl-acétal (ou tropital).

Les composés de formule I présentent une excellente tolérance générale, et l'invention a donc également pour objet les produits de formule I, pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale I, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

**Exemple 1 : [1R,[1alpha,3alpha]] 2,2-diméthyl 3-(2-bromo éthényl) cyclopropane carboxylate de 2,3,4,5,6 penta fluorophényl méthyle.**

On introduit à 0° 5° C une solution renfermant 1,55 g de dicyclohexylcarbodiimide, 0.1 g de 4 diméthyl amino pyridine et 10 cm3 de chlorure de méthylène dans une solution renfermant 1,5 g d'acide [1R,-[1alpha,3alpha (Z)]] 2,2-diméthyl 3(2-bromoéthényl) cyclopropane carboxylique, 1,6 g d'alcool 2,3,4,5,6 pentafluorophényl méthylique et 60 cm3 de chlorure de méthylène. On maintient le mélange réactionnel pendant 1/4 heure à 0° C puis 1 heure à 20° C. On filtre, concentre et chromatographie le produit obtenu sur silice en éluant par le mélange hexane, éther isopropylique 95-5. On sèche et obtient le produit recherché.

$alpha_D = +43° \pm 2°5$ (c=0,5 % $CHCl_3$).

Préparation 1 :Acide 1Rcis 2,2-diméthyl 3-[2-bromo (Z)-éthényl] cyclopropane carboxylique et isomère E correspondant.

Stade A : ester terbutylique

On ajoute 36 g d'hydrure de tributylétain dans une solution renfermant 21,2 g de 1Rcis 2,2-diméthyl 3-(2-dibromo éthényl) cyclopropane carboxylate de terbutyl et 30 cm3 de benzène. On agite à 50° C pendant 1 heure 30 et 16 heures à 20° C. On élimine le benzène. On chromatographie le résidu obtenu sur silice en éluant par le mélange hexane, éther isopropylique 95-5. On obtient 7,38 g de produit recherché F 30° C isomère Z et 3,86 g de produit recherché F = 74° C isomère E.

Stade B : Acide 1Rcis (Z) 3(2-bromo éthényl 2,2-diméthyl cyclopropane carboxylique et isomère E correspondant.

Le produit Z a été préparé par hydrolyse de l'ester terbutylique correspondant.
Le produit E a été préparé par hydrolyse de l'ester terbutylique correspondant.

**Exemple 2 : [1R[1alpha,3alpha(Z)]] 2,2-diméthyl 3(2-bromoéthényl) cyclopropane carboxylate de 2,3,5,6 tétrafluoro phényl méthyle.**

$alpha_D$ = +42° 5 ± 2° 5 (c = 0,5 % $CHCl_3$).

**Exemple 3 : [1R[1alpha,3alpha(Z)]] 2,2-diméthyl 3(2-bromoéthényl) cyclopropane carboxylate de 4-méthyl 2,3,5,6 *tétrafluoro* phényl méthyle.**

$alpha_D$ = +40° ± 2° 5 (c = 0,5 % $CHCl_3$).

**Exemple 4 : [1R[1alpha,3alpha(Z)]] 2,2-diméthyl 3(2-bromoéthényl) cyclopropane carboxylate de 4-méthoxy 2,3,5,6 tétra fluoro phényl méthyle.**

$alpha_D$ = +38° ± 2° 5 (c = 0,55 % $CHCl_3$).

**Exemple 5 : [1R[1alpha,3alpha(E)]] 2,2-diméthyl 3(2-bromoéthényl) cyclopropane carboxylate de 2,3,4,5,6 pentafluoro phényl méthyle.**

$alpha_D$ = -43° ± 2° 5 (c = 0,5 % $CHCl_3$).

**Exemple 6 : [1R[1alpha,3alpha(E)]] 2,2-diméthyl 3(2-bromoéthényl) cyclopropane carboxylate de 4-méthoxy 2,3,5,6 tétrafluoro phényl méthyle.**

$alpha_D$ = -52° 5 ± 1° 5 (c = 0,95 % $CHCl_3$).

**Exemple 7 : [1R[1alpha,3alpha(E)]] 3(2-bromoéthényl) 2,2-diméthyl cyclopropane carboxylate de 2,3,5,6 tétrafluoro phényl méthyle.**

$alpha_D$ = -48° 5 ± 1° 5 (c = 1 % $CHCl_3$).

**Exemple 8 à 12 :**

Les exemples suivants ont été préparés à partir des acides correspondants dont la préparation est donnée ci-après (préparation 2), et des alcools correspondants en suivant le mode opératoire de l'exemple 1.

**Exemple 8 : [1R[1alpha,3béta(Z)]] 3(2-bromoéthényl) 2,2-diméthyl cyclopropane carboxylate de**

**2,3,4,5,6 pentafluoro phényl méthyle.**

$alpha_D = -48^\circ \pm 2^\circ 5$ (c=0,5 % $CHCl_3$).

**Exemple 9 : [1R[1alpha,3béta(Z)]] 3(2-bromoéthényl) 2,2-diméthyl cyclopropane carboxylate de 2,3,5,6 tétrafluoro phényl méthyle.**

$alpha_D = -48^\circ \pm 2^\circ 5$ (c=0,5 % $CHCl_3$).

**Exemple 10 : [1R[1alpha,3béta(E)]] 3(2-bromoéthényl) 2,2-diméthyl cyclopropane carboxylate de 2,3,5,6 tétrafluoro phényl méthyle.**

$alpha_D = +27^\circ \pm 2^\circ$ (c=0,55 % $CHCl_3$).

**Exemple 11 : [1R[1alpha,3béta(E)]] 3(2-bromoéthényl) 2,2-diméthyl cyclopropane carboxylate de 4-méthoxy 2,3,5,6 tétrafluoro phényl méthyle.**

$alpha_D = +25^\circ \pm 2^\circ$ (c=0,5 % $CHCl_3$).

**Exemple 12 : [1R[1alpha,3béta(E)]] 3(2-bromoéthényl) 2,2-diméthyl cyclopropane carboxylate de 2,3,4,5,6 pentafluoro phényl méthyle.**

$alpha_D = +27^\circ 5 \pm 2^\circ 5$ (c=0,5 % $CHCl_3$).

Préparation 2 : Acide [1R[1alpha,3béta(E)]] 3(2-bromoéthényl) 2,2-diméthyl cyclopropane carboxylique et isomère Z correspondant.

Stade A : [1R[1alpha,3béta(E)]] 3(2-bromo éthényl) 2,2-diméthyl cyclopropane carboxylate de terbutyle.

On introduit 34 ml d'hydrure de tributylétain dans une solution renfermant 21,8 g de 1R(1alpha,3béta) 3-(2,2-dibromoéthényl) 2,2-diméthyl cyclopropane carboxylate de terbutyle et 45 ml de toluène. On chauffe à 50°C pendant 2 heures 30 minutes. On laisse revenir la température à 20°C. On chromatographie le produit obtenu sur silice en éluant par le mélange hexane-acétate d'éthyle (95-05). On isole le produit obtenu que l'on chromatographie à nouveau. On obtient 5,9 d'isomère E et 6, g d'isomère Z.

| Spectre RMN | | |
|---|---|---|
| | Isomère E | Isomère Z |
| $CH_3$ géminés | 1,22 (s) | 1,19 ; 1,29 |
| H en 3 du cyclopropane | 1,97 (dd,J=5,5 et 9) | 2,28 (m) |
| H en 1 du groupe éthényle | 5,91 (dd,J=9 et 13,5) | 5,84 (dd,J=7 et 8) |
| H en 2 du groupe éthényle | 6,15 (d,J=13,5) | 6,26 (dd,J=7 et 1) |
| H du tert-butyle | 1,45 (s) | 1,46 (s) |
| H en 1 du cyclopropane | 1,53 (d,J=13,5) | 1,53 (d,J=5,5) |

Stade B :

a) Acide [1R[1alpha,3béta(E)]] 3(2-bromo éthényl) 2,2-diméthyl cyclopropane carboxylique.

Le produit a été obtenu par clivage de l'ester correspondant.

Spectre RMN $CDCl_3$ :

H des $CH_3$ géminés 1,2 et 1,29 ppm
H en 1 du cyclopropane 1,61 (d,J = 5)
H en 3 du cyclopropane 2,09 (dd)
H en 1 du radical éthényl 5,91 (dd,J = 13,5 et 8,5)
H en 2 du radical éthényl 6,19 (d,J = 13,5)

b) Acide [1R[1alpha,3béta(Z)]] 3(2-bromo éthényl) 2,2-diméthyl cyclopropane carboxylique.

Le produit a été obtenu par clivage de l'ester correspondant F = 62° C.

Les produits des exemples 13 à 17 ont été préparés à partir des acides préparés comme indiqué à la préparation 3 ci-dessous, et des alcools correspondants en suivant le mode opératoire décrit à l'exemple 1.

**Exemple 13 : [1R[1alpha,3alpha(Z)]] 2,2-diméthyl 3(2-chloro éthényl) cyclopropane carboxylate de 4-méthoxy 2,3,5,6 tétrafluoro phényl méthyle.**

$alpha_D$ = +36° ± 1°5 (c = 1 % $CHCl_3$).

**Exemple 14 : [1R[1alpha,3alpha(Z)]] 2,2-diméthyl 3(2-chloro éthényl) cyclopropane carboxylate de 2,3,5,6 tétrafluoro phényl méthyle.**

$alpha_D$ = +44° ± 2° (c = 0,5 % $CHCl_3$).

**Exemple 15 : [1R[1alpha,3alpha(Z)]] 2,2-diméthyl 3(2-chloro éthényl) cyclopropane carboxylate de 4-méthyl 2,3,5,6 tétrafluoro phényl méthyle.**

$alpha_D$ = +42°5 ± 2°5 (c = 0,5 % $CHCl_3$).

**Exemple 16 : [1R[1alpha,3alpha(Z)]] 2,2-diméthyl 3(2-chloro éthényl) cyclopropane carboxylate de 2,3,4,5,6 pentafluoro phényl méthyle.**

$alpha_D$ = +42° ± 2°5 (c = 0,5 % $CHCl_3$).

**Exemple 17 : [1R[1alpha,3alpha(E)]] 3(2-chloro éthényl) 2,2-diméthyl cyclopropane carboxylate de 2,3,4,5,6 pentafluoro phényl méthyle.**

$alpha_D$ = -45° ± 2° (c = 0,5 % $CHCl_3$).

Préparation 3 : Acide [1R[1alpha,3alpha(Z)]] 2,2-diméthyl 3(2-chloro éthényl) cyclopropane carboxylique.

On introduit 49,8 g de chlorure de chlorométhyl triphényl phosphonium, dans une suspension renfermant de 15 g de lactone de l'acide 1R,cis 2,2-diméthyl (dihydroxy méthyl) cyclopropane carboxylique et 150 cm3 de tétrahydrofuran. On maintient la suspension obtenue pendant 1/2 heure à 0° C. On la refroidit à -65° C et introduit une solution renfermant 30 g de terbutylate de potassium et 300 cm3 de tétrahydrofuran. On agite pendant 1 heure à -65° C et laisse remonter la température jusqu'à 15° C. On obtient une solution

que l'on verse sur une solution aqueuse glacée de phosphate acide de sodium, extrait à l'éther isopropylique, lave à l'eau, sèche, filtre et concentre. On chromatographie le produit obtenu sur silice en éluant par le mélange hexane, acétate d'éthyle, acide acétique 7-3-0,1. On élimine le solvant, reprend au toluène pour éliminer l'acide acétique. On sèche et obtient à partir du mélange Z et E (85-15) l'isomère Z attendu, le produit recherché.

RMN $CDCl_3$

$CH_3$ géminés 1,28 ppm
H en 1 du cyclopropane 1,83 (d,J) = 8,5 ppm
H en 3 du cyclopropane 2,33 (dd,J) = 8,5 et 8 ppm
H en 1 et 2 du radical éthényle 6,09 à 6,3.

Acide [1R[1alpha,3alpha(E)]] 2,2-diméthyl 3(2-chloro éthényl) cyclopropane carboxylique.

Le produit a été préparé selon le schéma :

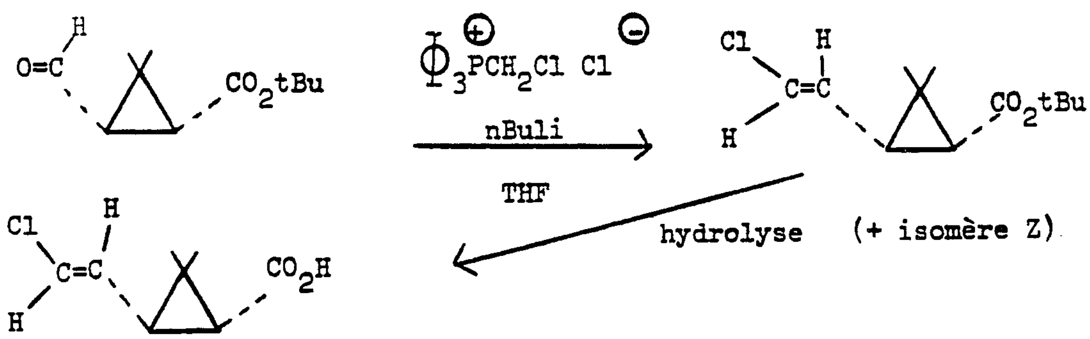


Les produits des exemples 18 à 24 ont été préparés à partir des acides correspondants préparés selon la préparation 4 et des alcools correspondants, en suivant le mode opératoire de l'exemple 1.

**Exemple 18 : [1R[1alpha,3béta(E)]] 3(2-chloro éthényl) 2,2-diméthyl cyclopropane carboxylate de 2,3,4,5,6 pentafluoro phényl méthyle.**

$alpha_D$ = +43°5 ± 2°5 (c = 0,4 % $CHCl_3$).

**Exemple 19 : [1R[1alpha,3béta(E)]] 3(2-chloro éthényl) 2,2-diméthyl cyclopropane carboxylate de 2,3,5,6 tétrafluoro phényl méthyle.**

$alpha_D$ = +17 ± 1° (c = 1 % $CHCl_3$).

**Exemple 20 : [1R[1alpha,3béta(E)]] 3(2-chloro éthényl) 2,2-diméthyl cyclopropane carboxylate de 4-méthoxy 2,3,5,6 tétrafluoro phényl méthyle.**

$alpha_D$ = +8°5 ± 2° (c = 0,6 % $CHCl_3$).

**Exemple 21 : [1R[1alpha,3béta(Z)]] 3(2-chloro éthényl) 2,2-diméthyl cyclopropane carboxylate de 2,3,4,5,6 pentafluoro phényl méthyle.**

$alpha_D$ = -26° ± 2° (c = 0,5 % $CHCl_3$).

**Exemple 22 : [1R[1alpha,3béta(Z)]] 3(2-chloro éthényl) 2,2-diméthyl cyclopropane carboxylate de 2,3,5,6 tétrafluoro phényl méthyle.**

alpha$_D$ = -33°5 ± 2° (c = 0,5 % $CHCl_3$).

**Exemple 23 : [1R[1alpha,3béta(Z)]] 3(2-chloro éthényl) 2,2-diméthyl cyclopropane carboxylate de 4-méthoxy 2,3,5,6 tétrafluoro phényl méthyle.**

alpha$_D$ = -44° ± 2° (c = 0,7 % $CHCl_3$).

Préparation 4 : Acide [1R[1alpha,3béta(E)]] 3(2-chloro éthényl) 2,2-diméthyl cyclopropane carboxylique et isomère Z correspondant.

Stade A : [1R[1alpha,3béta(E)]] 3(2-chloro éthényl) 2,2-diméthyl cyclopropane carboxylate de (1,1-diméthyl) éthyle et isomère Z correspondant.

Le produit a été obtenu à partir du mélange E + Z correspondant décrit dans le brevet français 2 185 612 par chromatographie sur silice en éluant par le mélange hexane-éther éthylique 97,3.

RMN ($CDCl_3$ ppm)

Isomère (E) :

H des méthyles 1,16-1,22
H du tert-butyl 1,45 ppm
H du carbone en 1 du cyclopropane 1,50 (d,J = 5,5)
H du carbone en 3 du cyclopropane 1,96 (dd,J = 5,5 et 9)
H du carbone en 1 du radical éthényl 5,66 (dd,J = 9 et 13)
H du carbone en 2 du radical éthényl 6,06 (d,J = 13).

Isomère (Z) :

H des méthyles 1,2-1,35
H du tert-butyle 1,45
H du carbone en 1 du cyclopropane 1,55-1,65
H du carbone en 3 du cyclopropane 2,23 à 2,65
H du carbone en 1 du radical éthényle 5,4 à 5,63
H du carbone en 2 du radical éthényle 6,1 à 6,25

Stade B : Acide [1R[1alpha,3béta(E)]] 3(2-chloro éthényl) 2,2- diméthyl cyclopropane carboxylique et isomère Z correspondant.

a) On maintient pendant 1 heure 30 au reflux, un mélange renfermant 3 g de produit préparé au Stade A isomère E, 30 cm3 de toluène et 0,15 g d'APTS. On laisse revenir à la température ambiante, concentre, dilue au chlorure de méthylène, lave à l'eau, sèche sur sulfate de sodium, filtre et concentre. On obtient 2,05 g de produit recherché.

b) en opérant comme ci-dessus à partir du produit de départ Z correspondant, on obtient le produit recherché F 50°C.

Les produits des exemples 24 à 26 ont été préparés à partir de l'acide préparé ci-après dans la préparation 5 et des alcools correspondants en suivant le mode opératoire de l'exemple 1.

**Exemple 24 : [1R(1alpha,3alpha(E+Z))] 2,2-diméthyl 3(2-fluoro éthényl) cyclopropane carboxylate de 2,3,4,5,6 pentafluoro phényl méthyle.**

F 30 °C.

**Exemple 25 : [1R(1alpha,3alpha(E+Z))] 2,2-diméthyl 3(2-fluoro éthényl) cyclopropane carboxylate de 2,3,5,6 tétrafluoro phényl méthyle.**

$alpha_D$ = 0 (c = $CHCl_3$).

**Exemple 26 : [1R(1alpha,3alpha(E+Z))] 2,2-diméthyl 3(2-fluoro éthényl) cyclopropane carboxylate de 4-méthoxy 2,3,5,6 tétrafluorophényl méthyle.**

$alpha_D$ = -6° +1° (c = 1 % $CHCl_3$).

Préparation 5 : Acide [1R(1alpha,3alpha(E+Z))] 2,2-diméthyl 3-(2-fluoro éthényl) cyclopropane carboxylique.

Stade A : [1R(1alpha,3alpha(R))] 2,2-diméthyl 3(1-hydroxy 2,2-dibromo 2-fluoro éthyl) cyclopropane carboxylate de terbutyle.

Le produit a été préparé selon le procédé décrit pour le procédé correspondant en série 1R-(1alpha,3béta).

Spectre RMN $CDCL_3$ ppm

gem méthyle 1,21 ; 1,28
H en 1 1,70 (d,J = 8,5)
OH 3,61 (d,J = 5)
H du carbone en 1 de
 OH
 |
- CH - 4,33 ddd,J = 5,8 et 9,5.

Stade B : [1R(1alpha,3alpha(E+Z))] 2,2-diméthyl 3-(2-fluoro éthényl) cyclopropane carboxylate de terbutyle.

Le produit a été préparé selon le procédé décrit pour le produit correspondant en série 1R-(1alpha,3béta).

Spectre IR dans $CHCl_3$

 O
 ||
- C - à 1712 $cm^{-1}$
-C = C à 1668 $cm^{-1}$
$CH_3$ de tbu à 1368 $cm^{-1}$

Acide [1R(1alpha,3alpha(E+Z))] 2,2-diméthyl 3(2-fluoro éthényl) cyclopropane carboxylique.

Le produit a été préparé par clivage de l'ester terbutylique correspondant.

Spectre IR

| | |
|---|---|
| C=O | 1734 $cm^{-1}$ |
| | 1698 $cm^{-1}$ |
| C=C | à 1670 $cm^{-1}$ |

Les produits des exemples 27 à 29 ont étà préparés selon le mode opératoire décrit à l'exemple 1, à partir de l'acide préparé selon la préparation 6, et des alcools correspondants.

**Exemple 27 : [1R(1alpha,3béta(E+Z))] 2,2-diméthyl 3(2-fluoro éthényl) cyclopropane carboxylate de 2,3,4,5,6 pentafluoro phényl méthyle.**

$alpha_D$ = -21° 5 ± 1° (c=1 % $CHCl_3$).

**Exemple 28 : [1R(1alpha,3béta(E+Z))] 2,2-diméthyl 3(2-fluoro éthényl) cyclopropane carboxylate de 4-méthoxy 2,3,5,6 tétrafluorophényl méthyle.**

$alpha_D$ = -20° 5 ± 2° (c=0,5 % $CHCl_3$).

**Exemple 29 : [1R(1alpha,3béta(E+Z))] 2,2-diméthyl 3(2-fluoro éthényl) cyclopropane carboxylate de 2,3,5,6 tétrafluoro phényl méthyle.**

$alpha_D$ = -22° 5 ± 1 (c=1 % $CHCl_3$).

Préparation 6 : Acide [1R(1alpha,3béta(E+Z))] 2,2-diméthyl 3(2-fluoro éthényl) cyclopropane carboxylique.

Stade A : [1R(1alpha,3béta(R+S))] 2,2-diméthyl 3(1-hydroxy 2,2-dibromo 2-fluoro éthyl) cyclopropane carboxylate de terbutyle.

On ajoute à 0° C, 2 ml de fluoro dibromo méthane dans une solution renfermant 4,7 g de 1R-(1alpha,3béta) 2,2-diméthyl 3-formyl cyclopropane carboxylate de terbutyle et 50 ml de tétrahydrofuranne. On refroidit le mélange réactionnel à -65° C et ajoute goutte à goutte en 45 minutes 2,8 g de terbutylate de potassium en solution dans 20 ml de tétra- hydrofuranne. On agite pendant 40 minutes à -65° C le milieu réactionnel. On verse le milieu réactionnel sur 100 ml de phosphate acide de sodium. On extrait à l'acétate d'éthyle, on sèche et concentre. On chromatographie sur silice en éluant par le mélange hexane acétate d'éthyle 8-2 F 110° C.

Stade B : [1R(1alpha,3béta(E+Z))] 2,2-diméthyl 3(2-fluoro éthényl) cyclopropane carboxylate de terbutyle.

On mélange sous pression d'azote, 6,1 g de produit préparé au Stade A dans une solution renfermant 40 ml d'acide acétique et 4 ml d'eau. On ajoute à 10° C, 4 g de zinc en poudre. On maintient le mélange réactionnel sous agitation pendant 2 heures. On ajoute 300 ml d'éther isopropylique et filtre. On lave à l'eau. On sèche et concentre. On obtient 4 g de produit brut que l'on chromatographie sur silice en éluant par le mélange hexane acétate d'éthyle 9-1. On isole 2,4 g d'ester E + Z que l'on utilise tel quel dans le stade suivant.

Stade C : Acide [1R(1alpha,3béta(E+Z))] 2,2-diméthyl 3(2-fluoro éthényl) cyclopropane carboxylique.

On ajoute 150 mg d'acide paratoluène sulfonique dans une solution renfermant 2,39 g de produit obtenu au Stade B et 30 ml de toluène. On chauffe le milieu réactionnel au reflux pendant 3 heures. On chromatographie le produit obtenu sur silice en éluant par le mélange hexane acétate d'éthyle (1-1). On obtient 1,5 g de produit recherché.

Spectre RMN $CDCl_3$

| | Isomère (Z) | Isomère (E) |
|---|---|---|
| $CH_3$gem | 1,18 (s) | 1,16 (s) |
| | 1,32 (s) | 1,29 (s) |
| H en 1 et en 3 | 1,53 (d,J=5,5) | 1,47 (d,J=5,5) |
| | 2,36 (dd,J=5,5 et 9) | 1,94 (dd,J=5,5 et 8,5) |

| | |
|---|---|
| 4,53 (ddd,J=41,9 et 5)<br>6,58 (dd,J=84 et 5) 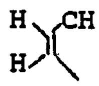 | 5,22(ddd,J=18,11 et 8,5)<br>6,64 (dd,J=84 et 11) 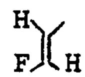 |

Le produit de l'exemple 30 a été préparé à partir de mélange d'isomères E + Z de l'acide obtenu à la préparation 3 (isomère Z majoritaire) avec l'alcool correspondant en utilisant le mode opératoire de l'exemple 1.

**Exemple 30 : 1R [1alpha, 3alpha (E + Z)] 2,2-diméthyl 3-(2-chloroéthényl) cyclopropane carboxylate de 1-(2-propynyl)-2,3,5,6-tétrafluorophénylméthyle.** (Spectre IR ($CHCl_3$) :

>C = O : 1728 $cm^{-1}$
-C = C- : 1628 $cm^{-1}$
aromatique : 1494 $cm^{-1}$
-C≡CH : 3308 F

<u>Spectre RMN (CDCl$_3$) 250 MHz)</u> :

<u>Isomère Z</u> :

| | | |
|---|---|---|
| cyclopropyle cis | 1,83 (d) = 8,5 Hz | : $H_1$ |
| | 2,28 (dd) = 8,5 et 8 Hz | : $H_3$ |
| 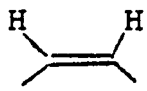 | 6,12 à 6,18 | |

<u>Isomère E</u> :

| | | |
|---|---|---|
| cyclopropyle cis | 1,73 (d) = 8 Hz | : $H_1$ |
| | 1,84 | : $H_3$ |
| 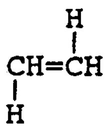 | 6,06 (d) = 13,5 Hz<br>6,24 (dd) = 13,5 et 10 Hz H | |

<u>Isomères E et Z</u> :

| | |
|---|---|
| $CH_3$ géminés | 1,26 - 1,27<br>1,28 |
| HC≡C- | : 2,07 (t) = 3 Hz |
| C≡C-$CH_2$ | : 3,64 (m) |
| 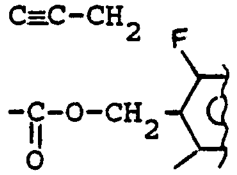 | : 5,20 (m) |

En opérant comme pour les exemples précédents à partir de l'acide 2 et de l'alcool appropriés, on a obtenu les produits des exemples suivants :

<u>**Exemple 31 : [1R (1alpha, 3béta) (Z))] 3-(2-bromoéthényl) 2,2-diméthyl cyclopropane carboxylate de 4-méthoxy 2,3,5,6-tétrafluorophénylméthyle.**</u>

[alpha]$_D$ = -37,5° ± 2° (c = 0,5% CHCl$_3$).

<u>**Exemple 32 : [1R (1alpha, 3alpha) (E))] 3-(2-chloroéthényl) 2,2-diméthyl cyclopropane carboxylate de 2,3,5,6-tétrafluorophénylméthyle.**</u>

[alpha]$_D$ = -33,5° ± 2,5° (c = 0,5% CHCl$_3$).

<u>**Exemple 33 : [1R (1alpha, 3alpha) (E))] 3-(2-chloroéthényl) 2,2-diméthyl cyclopropane carboxylate de 4-méthyl 2,3,5,6-tétrafluorophénylméthyle.**</u>

[alpha]$_D$ = -30° ± 2,5° (c = 0,5% CHCl$_3$).

**Exemple 34 : [1R (1alpha, 3alpha) (Z))] 2,2-diméthyl 3-(2-chloroéthényl) 2,2-diméthyl cyclopropane carboxylate de 4-éthyl 2,3,5,6-tétrafluorophénylméthyle.**

$[alpha]_D$ = + 24,5° ± 2° (c = 0,4% $CHCl_3$).

**Exemple 35 : 1Rcis 2,2-diméthyl 3-(delta Z 2-chloroéthényl) cyclopropane carboxylate de 4-éthyl 2,3,5,6-tétrafluorophénylméthyle.**

Rf : 0,27 (éluant : hexane-éther isopropylique 95-05).

**Exemple 36 : 1Rcis 2,2-diméthyl 3-(delta E 2-chloroéthényl cyclopropane carboxylate de 4-triméthylsilyl 2,3,5,6-tétrafluorophénylméthyle.**

Rf : 0,25 (éluant : hexane-éther isopropylique 95-05).

**Exemple 37 : [1R (1alpha, 3alpha) (Z))] 3-(2-chloroéthényl) 2,2-diméthyl cyclopropane carboxylate de 4-trifluorométhyl 2,3,5,6-tétrafluorophénylméthyle.**

$[alpha]_D$ = + 42° ± 2,5° (c = 0,3% $CHCl_3$).

**Exemple 38 : 1Rcis 2,2-diméthyl 3-[(Z) 2-chloroéthényl) cyclopropane carboxylate de 4-difluorométhyl 2,3,5,6-tétrafluorophénylméthyle.**

Rf : 0,18 (éluant : hexane-éther isopropylique 95-05).

**Exemple 39 : 1Rcis 2,2-diméthyl 3-[(delta E) 2-chloroéthényl) cyclopropane carboxylate de 4-difluorométhoxy 2,3,5,6-tétrafluorophénylméthyle.**

Rf : 0,17 (éluant : hexane-éther isopropylique 95-05).

**Exemple 40 : [1R (1alpha, 3alpha) (Z))] 2,2-diméthyl 3-(2-chloroéthényl) cyclopropane carboxylate de 4-méthoxyméthyl 2,3,5,6-tétrafluorophénylméthyle.**

$[alpha]_D$ = + 43,5° ± 1,5° (c = 0,8% $CHCl_3$).

**Exemple 41 : [1R (1alpha, 3alpha) (E))] 3-(2-chloroéthényl) 2,2-diméthyl cyclopropane carboxylate de 4-méthoxyméthyl 2,3,5,6-tétrafluorophénylméthyle.**

$[alpha]_D$ = + 39,5° ± 2,5° (c = 0,5% $CHCl_3$).

**Exemple 42 : 1Rcis 2,2-diméthyl 3-[(delta Z) 2-chloroéthényl)] cyclopropane carboxylate de 4-éthényl 2,3,5,6-tétrafluorophénylméthyle.**

Rf : 0,18 (éluant : hexane-éther isopropylique 98-02).

**Exemple 43 : 1Rcis 2,2-diméthyl 3-[(delta E) 2-chloroéthényl)] cyclopropane carboxylate de 4-éthényl 2,3,5,6-tétrafluorophénylméthyle.**

Rf : 0,17 (éluant : hexane-éther isopropylique 97-03).

**Exemple 44 : 1Rcis 2,2-diméthyl 3-[(delta Z) 2-chloroéthényl)] cyclopropane carboxylate de 4-éthynyl 2,3,5,6-tétrafluorophénylméthyle.**

Rf : 0,25 (éluant : hexane-éther isopropylique 97-03).

**Exemple 45 : 1Rcis 2,2-diméthyl 3-[2-chloroéthényl] cyclopropane carboxylate de 4-(2-propynyl) 2,3,5,6-tétrafluorophénylméthyle (75% d'isomère (delta Z), 25% d'isomère (delta E).**

$[alpha]_D = + 16^\circ \pm 1,5^\circ$ (c = 0,7% $CHCl_3$).

**Exemple 46 : 1Rcis 2,2-diméthyl 3-[(Z) 2-fluoroéthényl] cyclopropane carboxylate de 4-propynyl 2,3,5,6-tétrafluorophénylméthyle.**

$[alpha]_D = + 10^\circ \pm 1^\circ$ (c = 1% $CHCl_3$).

**Exemple 47 : 1Rcis 2,2-diméthyl 3-[(E) 2-fluoroéthényl] cyclopropane carboxylate de 4-propynyl 2,3,5,6-tétrafluorophénylméthyle.**

$[alpha]_D = - 10^\circ \pm 1^\circ$ (c = 1% $CHCl_3$).

**Exemple 48 : [1R (1alpha, 3alpha) (Z))] 3-(2-chloroéthényl) 2,2-diméthyl cyclopropane carboxylate de 3,4,5,6-tétrafluorophénylméthyle.**

$[alpha]_D = + 71^\circ \pm 2,5^\circ$ (c = 0,3% toluène).

**Exemple 49 : 1Rcis 2,2-diméthyl 3-[(Z) 2-fluoroéthényl] cyclopropane carboxylate de 4-propynyl 2,3,5,6-tétrafluorophénylméthyle.**

F < 50° C. Rf : 0,16 (éluant : hexane-acétate d'éthyle 98-02).

**Exemple 50 : Préparation d'un concentré soluble**

On effectue un mélange homogène de :
Produit de l'exemple 1 : 0,25 g
Butoxyde pipéronyle : 1,00 g
Tween 80 : 0,25 g
Topanol A : 0,1 g
Eau : 98,4 g

**Exemple 51 : Préparation d'un concentré émulsifiable**

On mélange intimement :
Produit de l'exemple 2 : 0,015 g
Butoxyde de pipéronyle : 0,5 g
Topanol A : 0,1 g
Tween 80 : 3,5 g
Xylène : 95,885 g

**Exemple 52 : Préparation d'un concentré émulsifiable**

On effectue un mélange homogène de :

Produit de l'exemple 3 : 1,5 g
Tween 80 : 20,00 g
Topanol A : 0,1 g
Xylène : 78,4 g

**Exemple 53 : Préparation d'une composition fumigène**

On mélange d'un façon homogène :

Produit de l'exemple 14 : 0,25 g
Poudre de tabu : 25,00 g
Poudre de feuille de cèdre : 40,00 g
Poudre de bois de pin : 33,75 g
Vert brillant : 0,5 g
p-Nitrophénol : 0,5 g

## ETUDE BIOLOGIQUE

A. Activité sur DIABROTICA

Les insectes tests sont des larves de dernier stade de Diabrotica.

On traite une rondelle de papier filtre de 9 cm de diamètre, déposée au fond d'une boite de Pétri, à l'aide de 2 $cm^3$ de solution acétonique. Après séchage on dépose 10 larves par dose et on effectue le contrôle de mortalité 24 heures après le traitement.On détermine la dose létale 100 (DL 100) exprimée en mg/litre.

Les résultats obtenus sont les suivants :

Exemple 1 : 2,5
Exemple 2 : 2,5
Exemple 3 : 1,2
Exemple 4 : 5
Exemple 5 : 5
Exemple 13 : 5
Exemple 14 : 1,2
Exemple 15 : 1,2
Exemple 16 : 1,2
Exemple 17 : 1,2
Exemple 18 : 5
Exemple 24 : 1,2
Exemple 25 : 2,5
Exemple 26 : 2,5
Exemple 30 : 0,6
Exemple 46 : 0,3
Exemple 47 : 1,2
Exemple 49 : 0,3

B. Etude de l'effet d'abattage sur mouche domestique

Les insectes tests sont des mouches domestiques femelles âgées de 4 jours. On opère par pulvérisation en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Composés | $KT_{50}$ en mn | Concentration en g/l |
|---|---|---|
| Ex. 1 | 2,5 | 1 |
| Ex. 2 | 2,5 | 1 |
| Ex. 5 | 2,2 | 1 |
| Ex. 6 | 3 | 1 |
| Ex. 13 | 2,3 | 1 |
| Ex. 16 | 2,9 | 1 |
| Ex. 17 | 9,1 | 0,1 |
| Ex. 24 | 7,6 | 0,1 |
| Ex. 25 | 7,8 | 0,1 |
| Ex. 30 | 5 | 0,1 |
| Ex. 39 | 6,7 | 0,1 |
| Ex. 40 | 5,1 | 0,1 |
| Ex. 41 | 3,1 | 0,1 |
| Ex. 46 | 3,2 | 0,1 |
| Ex. 47 | 1,5 | 0,1 |
| Ex. 49 | 3,3 | 0,1 |

C) Etude de l'effect létal sur mouche domestique

Les insectes tests sont des mouches domestiques femelles âgées de 4 à 5 jours. On opère par application topique de 1 microlitre de solution acétonique sur le thorax dorsal des insectes à l'aide du micro manipulateur d'Arnold. On utilise 50 individus par traitement. On effectue le contrôle de mortalité vingt-quatre heures après traitement.

Les résultats obtenus exprimés en $DL_{50}$ ou dose (en nanogrammes) par individu nécessaire pour tuer 50 % des insectes, sont les suivants :

| Composés de l'exemple | DL 50 en ng/insecte |
|---|---|
| 45 | 8,2 |
| 47 | 2,5 |
| 49 | 2,5 |

D) Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24° C et 65 % d'humidité relative. Après traitement les individus sont placés sur milieu nutritif artificiel (milieu de Poitout).

On effectue le contrôle des mortalités 48 heures après traitement.

Les résultats expérimentaux obtenus sont résumés dans le tableau suivant :

| Composés de l'exemple | DL 50 en ng par insecte |
|---|---|
| 49 | 2,7 |

D) Etude de l'effet létal sur Aphis cracivora.

On utilise des adultes après 7 jouts et l'on emploie 10 Aphis par concentration utilisée. On utilise une méthode de contact-injection. On effectue le traitement au pistolet de Fisher d'une feuille de fève que l'on dépose dans une boîte de Pétri en matière plastique sur une rondelle de papier humidifiée. Le traitement est effectué à l'aide de 2 ml de solution acétonique de produit à tester (1ml par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composés de l'exemple | CL 50 en mg/l |
|---|---|
| 45 | 0,2 |
| 46 | 0,7 |
| 47 | 0,2 |
| 49 | 0,3 |

**Revendications**

1) Sous toutes les formes stéréoisomères possibles, ainsi que les mélanges de ces stéréoisomères les composés de formule (I) :

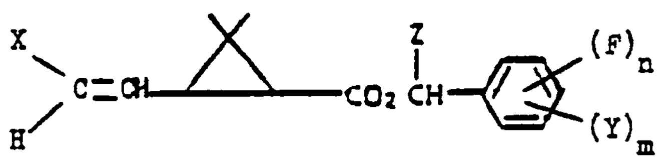

(I)

dans laquelle :

- X représente un atome d'halogène ;
- Z représente un atome d'hydrogène, un radical $CH_3$, C≡N ou C≡CH ;
- n représente un nombre entier pouvant varier de 1 à 5 ;
- m représente le nombre 5-n ;
- Y représente un atome d'hydrogène, un atome d'halogène, un radical $CH_2$ -C≡N, un radical hydroxy, un radical alkyle linéaire ou ramifié, saturé ou insaturé éventuellement substitué renfermant jusqu'à 8 atomes de carbone, un radical nitrile un radical

O
‖
C -O-alkyle,

O
‖
C -alkyle, $(CH_2)m'$ O-alkyle, $(CH_2)m'$ S alkyle, $(CH_2)m'$ N-$(alkyle)_2$, dans lequel m' représente le nombre 0, 1, 2, 3 ou 4 et renfermant jusqu'à 12 atomes de carbone un radical Si $(alkyle)_3$, alkyle représente un radical

alkyle linéaire ou ramifié, saturé ou insaturé, éventuellement substitué renfermant jusqu'à 8 atomes de carbone, un radical O-aryle ou $-(CH_2)m'$-aryle, aryle représentant un radical aryle renfermant jusqu'à 14 atomes de carbone, le ou les substituants Y identiques ou différents, pouvant être en position quelconque sur le noyau phényle.

2) Les composés de formule (I) tels que définis à la revendication 1 répondant à la formule ($I_A$) :

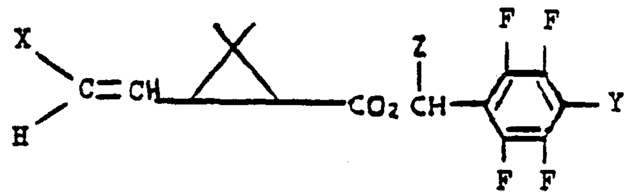

($I_A$)

dans lesquels X, Z et Y conserve la même signification que dans la revendication 1.

3) Les composés de formule ($I_A$) tels que définis à la revendication 2 dans lesquels :
- Y représente un atome d'hydrogène, un atome d'halogène, un radical hydroxy un radical alkyle linéaire ou ramifié, saturé ou insaturé éventuellement substitué renfermant jusqu'à 8 atomes de carbone, un radical nitrile, un radical $(CH_2)m'$ O-alkyle, $(CH_2)m'$ S alkyle, $(CH_2)m'$ N-$(alkyle)_2$, dans lequel m' représente le nombre 0, 1, 2, 3 ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou $-(CH_2)m'$-aryle, aryle représentant un radical aryle renfermant jusqu'à 14 atomes de carbone.

4) Les composés de formule (I) ou ($I_A$) tels que définis à l'une quelconque des revendications 1 à 3 dans lesquels Z représente un atome d'hydrogène.

5) Les composés de formule (I) ou ($I_A$) tels que définis à l'une quelconque des revendications 1 à 4 dans lesquels X représente un atome de fluor, de chlore ou de brome.

6) Les composés de formule (I) ou ($I_A$) tels que définis à l'une quelconque des revendications 1 à 5 dans lesquels Y représente un atome d'hydrogène.

7) Les composés de formule (I) ou ($I_A$) tels que définis à l'une quelconque des revendications 1 à 5 dans lesquels Y représente un atome de fluor.

8) Les composés de formule (I) ou ($I_A$) tels que définis à l'une quelconque des revendications 1 à 5 dans lesquels Y représente un radical alkyle linéaire saturé ou insaturé renfermant jusqu'à 4 atomes de carbone.

9) Les composés de formule (I) ou ($I_A$) tels que définis à la revendication 8 dans lesquels Y représente un radical méthyle.

10) Les composés de formule (I) ou ($I_A$) tels que définis à la revendication 8, dans lesquels Y représente un radical propynyle.

11) Les composés de formule (I) ou ($I_A$) tels que définis à l'une quelconque des revendications 1 à 10 dans lesquels la copule cyclopropanique est de structure 1R cis.

12) Les composés de formule ($I_A$) définis à la revendication 2 dont les noms suivent :
- le [1R[1alpha,3alpha(Z)]] 2,2-diméthyl 3(2-chloro éthényl) cyclopropane carboxylate de 2,3,5,6 tétrafluoro phényl méthyle ;
- le [1R[1alpha,3alpha(Z)]] 2,2-diméthyl 3(2-bromo éthényl) cyclopropane carboxylate de 2,3,4,5,6 pentafluoro phényl méthyle ;
- le [1R[1alpha,3alpha(Z)]] 2,2-diméthyl 3(2-bromo éthényl) cyclopropane carboxylate de 2,3,5,6 tétrafluoro phényl méthyle ;
- le [1R[1alpha,3alpha(Z)]] 2,2-diméthyl 3(2-bromo éthényl) cyclopropane carboxylate de 4-méthyl 2,3,5,6 tétrafluoro phényl méthyle,
- le [1R[1alpha,3alpha(E + Z)]] 2,2-diméthyl 3-(2-chloroéthényl) cyclopropane carboxylate de 4-(2-propynyl) 2,3,5,6-tétrafluoro phényl méthyle,
- le [1R[1alpha,3alpha(Z)]] 2,2-diméthyl 3-(2-fluoroéthényl) cyclopropane carboxylate de 4-(2-propynyl) 2,3,5,6-tétrafluoro phényl méthyle,
- le [1R[1alpha,3alpha(E)]] 2,2-diméthyl 3-(2-fluoroéthényl) cyclopropane carboxylate de 4-(2-propynyl) 2,3,5,6-tétrafluoro phényl méthyle,
- le [1R[1alpha,3alpha(Z)]] 2,2-diméthyl 3-(2-chloroéthényl) cyclopropane carboxylate de 4-(2-propynyl) 2,3,5,6-tétrafluoro phényl méthyle.

13) Procédé de préparation des composés de formule (I) tels que définis à l'une quelconque des revendications 1 à 12, caractérisé en ce que l'on soumet en présence d'un agent d'estérification un acide de formule :

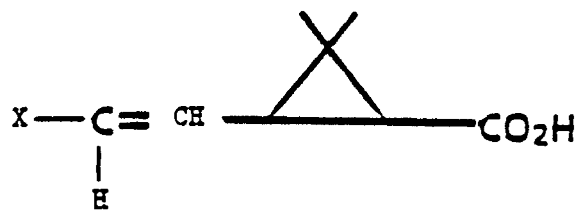

(II)

dans laquelle X conserve sa signification précédente à l'action d'un alcool de formule :

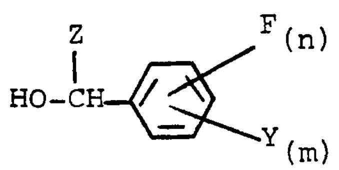

(III)

dans laquelle Y, Z, n et m conservent leur signification précédente pour obtenir le composé de formule (I) correspondant.

14) Application des composés de formule (I) ou ($I_A$) tels que définis à l'une quelconque des revendications 1 à 10, à la lutte contre les parasites des végétaux et les parasites des locaux.

15) Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 12.

16) Les compositions insecticides, caractérisées en ce qu'elles renferment comme principe actif au moins un composé défini à l'une quelconque des revendications 1 à 12.

17) Les compositions insecticides définies à la revendication 16 caractérisées en ce qu'elles sont destinées à la lutte contre DIABROTICA et les autres parasites du sol.

18) Les compositions acaricides, caractérisées en ce qu'elles renferment comme principe actif au moins un composé défini à l'une quelconque des revendications 1 à 12.

19) Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3- tétrahydrothiophénylidène méthyl) cyclopropane-1-carboxyliques, par les esters d'alcool 3-phénoxy benzylique et d'alcools alpha- cyano 3-phénoxy benzyliques des acides 2,2-diméthyl 3-(2,2-dichlorovinyl) cyclopropane-1- carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2- isopropyl acétiques, par les esters d'alléthrolone, d'alcool 3,4,5,6- tétrahydrophtalimidométhylique, d'alcool 5-benzyl 3-furyl méthylique, d'alcool 3-phénoxy benzylique et d'alcool alpha-cyano 3-phénoxy benzyliques des acides 2,2-diméthyl 3-(-1,2,2,2-tétrahaloéthyl) cyclopropane-1- carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyrethrinoïdes ci-dessus.

20) A titre de produits intermédiaires nouveaux, les composés de formule (II) tels que définis à la revendication 13 dans lesquels X ou bien représente un atome de brome ou de fluor, ou bien représente un atome de chlore lorsque la copule cyclopropanique est de structure 1Rcis.

Revendications pour l'Etat contractant suivant : ES

1) Procédé de préparation des composés de formule (I) :

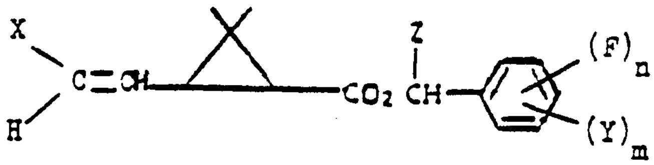

(I)

sous toutes les formes stéréoisomères possibles ainsi que les mélanges de ces stéréoisomères dans laquelle :

- X représente un atome d'halogène ;
- Z représente un atome d'hydrogène, un radical $CH_3$, C N ou C CH ;
- n représente un nombre entier pouvant varier de 1 à 5 ;
- m représente le nombre 5-n ;
- Y représente un atome d'hydrogène, un atome d'halogène, un radical $CH_2$ C N, un radical hydroxy, un radical alkyle linéaire ou ramifié, saturé ou insaturé éventuellement substitué renfermant jusqu'à 8 atomes de carbone, un radical nitrile un radical

O

C -O-alkyle,

O

C -alkyle, $(CH_2)m'$ O-alkyle, $(CH_2)m'$ S alkyle, $(CH_2)m'$ N-$(alkyle)_2$, dans lequel m' représente le nombre 0, 1, 2, 3 ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone, le ou les substituants Y identiques ou différents, pouvant être en position quelconque sur le noyau phényle, caractérisé en ce que l'on soumet en présence d'un agent d'estérification un acide de formule :

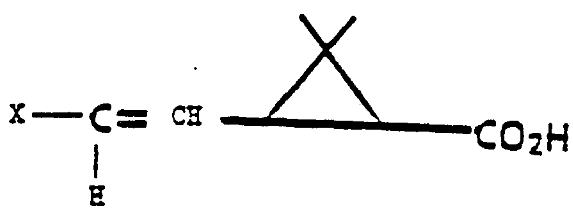

(II)

dans laquelle X conserve sa signification précédente à l'ac tion d'un alcool de formule :

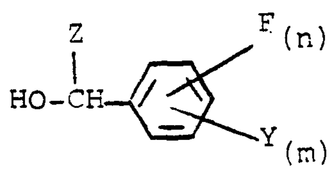

(III)

dans laquelle Y, Z, n et m conservant leur signification précédente pour obtenir le composé de formule (I) correspondant.

2) Procédé selon la revendication 1, caractérisé en ce que l'on utilise au départ un produit de formule (III) répondant à la formule :

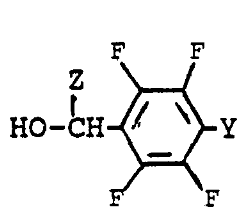

dans lesquels Y et Z conservent la même signification que dans la revendication 1.

3) Procédé selon la revendication 2, caractérisé en ce que dans le produit de formule (III) :

- Y représente un atome d'hydrogène, un atome d'halogène, un radical hydroxy un radical alkyle linéaire ou ramifié, saturé ou insaturé éventuellement substitué renfermant jusqu'à 8 atomes de carbone, un radical nitrile, un radical $(CH_2)m'$ O-alkyle, $(CH_2)m'$ S alkyle, $(CH_2)m'$ N-$(alkyle)_2$, dans lequel m′ représente le nombre 0, 1, 2, 3 ou 4 et renfermant jusqu'à 12 atomes de carbone un radical Si $(alkyle)_3$, alkyle représente un radical alkyle linéaire ou ramifié, saturé ou insaturé, éventuellement substitué renfermant jusqu'à 8 atomes de carbone, un radical O-aryle ou -$(CH_2)m'$-aryle, aryle représentant un radical aryle renfermant jusqu'à 14 atomes de carbone.

4) Procédé selon l'une quelconque des revendications 1 à 3, caractérisé en ce que l'on utilise au départ un produit de formule (III) dans lequel Z représente un atome d'hydrogène.

5) Procédé selon l'une quelconque des revendications 1 à 4, caractérisé en ce que l'on utilise au départ un produit de formule (II) dans lequel X représente un atome de fluor, de chlore ou de brome.

6) Procédé selon l'une quelconque des revendications 1 à 5, caractérisé en ce que l'on utilise au départ un produit de formule (III) dans lequel Y représente un atome d'hydrogène.

7) Procédé selon l'une quelconque des revendications 1 à 5, caractérisée en ce que l'on utilise au départ un produit de formule (III) dans lequel Y représente un atome de fluor.

8) Procédé selon l'une quelconque des revendications 1 à 5, caractérisé en ce que l'on utilise au départ un produit de formule (III) dans lequel Y représente un radical alkyle linéaire saturé ou insaturé renfermant jusqu'à 4 atomes de carbone.

9) Procédé selon la revendication 8, caractérisé en ce que dans le produit de formule (III), Y représente un radical méthyle.

10) Procédé selon la revendication 8, caractérisé en ce que dans le produit de formule (III), Y représente un radical propynyle.

11) Procédé selon l'une quelconque des revendications 1 à 10, caractérisé en ce que l'on utilise au départ un produit de formule (II) dans lequel la copule cyclopropanique est de structure 1R cis.

12) Procédé selon la revendication 2, caractérisé en ce que l'on utilise au départ l'acide [1R-[1alpha,3alpha(Z) ou (Z + E)]] 2,2-diméthyl 3-(2-chloro éthényl) cyclopropane carboxylique ou l'acide [1R-[1alpha,3alpha(Z)]] 2,2-diméthyl 3-(2-bromo éthényl) cyclopropane carboxylique ou l'acide [1R-[1alpha,3alpha(Z) ou (E)]] 2,2-diméthyl 3(2-fluoroéthényl) cyclopropane carboxylique et l'alcool 2,3,5,6 tétrafluorobenzylique ou l'alcool 2,3,4,5,6-pentafluorobenzylique ou l'alcool 4-méthyl 2,3,5,6-tétrafluorobenzylique ou l'alcool 4-(2-propynyl) 2,3,5,6-tétrafluorobenzylique.

Office européen des brevets

# RAPPORT DE RECHERCHE EUROPEENNE

Numero de la demande

EP 90 40 0229

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | EP-A-0 302 612 (IMPERIAL CHEMICAL INDUSTRIES PLC)<br>* page 12, exemple 3 * | 1,20 | C 07 C 69/743<br>C 07 C 61/40<br>A 01 N 53/00 |
| A | PESTICIDE SCIENCE<br>tome 17, no. 6, decembre 1986, pages 708-714, Oxford, GB; M. ELLIOTT et al.: "The Pyrethrins and Related Compounds. Part XXXa: Esters from Acids with Mono-Halovinyl Side Chains" * page 48, tableau 1 * | 1 | |
| X | idem | 20 | |
| L,A | CHEMICAL ABSTRACTS<br>tome 110, no. 17, 24 avril 1989, abrégé no. 149800h, Columbus, Ohio, US; J. QIAN et al.: "Photolysis of deltamethrin in solution" & Huanjing Kexue Xuebao 1988, vol. 8, no. 3, pages 275-285 & C.A. Formular Index 110 F3, page 2793F, colonne 1, lignes 38-48 | 1,20 | **DOMAINES TECHNIQUES RECHERCHES (Int. Cl.5)** |
| A | GB-A-1 438 129 (SHELL INTERNATIONALE RESEARCH)<br>* revendication 1 * | 1 | C 07 C 69/00<br>C 07 C 61/00<br>A 01 N 53/00 |
| A | DE-A-2 544 150 (KURARAY)<br>* revendications 1,16 * | 1 | |
| A | PATENT ABSTRACTS OF JAPAN<br>tome 1, no. 28 (1488)(C-76), 28 mars 1977; & JP - A - 51 146 440 (SAGAMI CHOU K.K.) 16.12.1976 | 1 | |
| A | FR-A-2 341 307 (ROUSSEL UCLAF)<br>* revendication 1 * | 1 | |

-/-

Le présent rapport a été établi pour toutes les revendications

| Lieu de la recherche | Date d'achèvement de la recherche | Examinateur |
|---|---|---|
| BERLIN | 09-04-1990 | PROBERT C.L. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 90 40 0229

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 024 163 (ELLIOTT)<br>* colonne 1, ligne 48 - colonne 2, ligne 3 *<br>--- | 1,20 | |
| A | DE-A-2 654 062 (BAYER)<br>* revendication 1 *<br>----- | 1,20 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| BERLIN | 09-04-1990 | PROBERT C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P0402)